Europäisches Patentamt

European Patent Office

Office européen des brevets

(11) Publication number: **0 266 707 B1**

## EUROPEAN PATENT SPECIFICATION

(45) Date of publication of patent specification: **22.01.92**  (51) Int. Cl.⁵: **A61K 9/22**, A61K 31/165

(21) Application number: **87116021.4**

(22) Date of filing: **31.10.87**

(54) **Sustained release labetalol tablet.**

(30) Priority: **03.11.86 US 926306**

(43) Date of publication of application:
**11.05.88 Bulletin 88/19**

(45) Publication of the grant of the patent:
**22.01.92 Bulletin 92/04**

(84) Designated Contracting States:
**AT BE CH DE ES FR GB GR IT LI LU NL SE**

(56) References cited:
**DE-A- 2 707 853**
**FR-A- 2 588 188**
**GB-A- 2 181 053**
**US-A- 4 012 444**

(73) Proprietor: **SCHERING CORPORATION**
**2000 Galloping Hill Road**
**Kenilworth New Jersey 07033(US)**

(72) Inventor: **Sangekar, Surendra**
**214 Riverside Drive New York**
**New York 10025(US)**
Inventor: **Vadino, Winston A.**
**9 Glenmont Road Whitehouse Station**
**New Jersey 08889(US)**

(74) Representative: **von Kreisler, Alek,**
**Dipl.-Chem. et al**
**Deichmannhaus am Hauptbahnhof**
**W-5000 Köln 1(DE)**

EP 0 266 707 B1

**Description**

The present invention relates to a sustained release preparation of labetalol. Specifically, it relates to an oral dosage form which provides a release period suitable for single daily dosing which exhibits good bioavailability.

Labetalol hydrochloride, 5-[1-hydroxy-2-(1-methyl-3-phenylpropyl)aminoethyl]salicylamide HCl, is an adrenergic receptor blocking agent that has both selective alpha$_1$- and non-selective beta-adrenergic receptor blocking action. This drug is effective in the management of hypertension. The conventional dosage form marketed is intended for twice daily administration. A key factor in the management of hypertension is patient compliance and one approach to improving patient compliance is to reduce the number of times the patient must take the medication during a given period.

Labetalol hydrochloride, though appreciably soluble in lower and higher pH solutions, shows minimum solubility between pH 6 to 10. Consequently although labetalol hydrochloride may be soluble and available for absorption in gastric pHs, it may not be available for absorption at intestinal pHs due to precipitation of labetalol hydrochloride in this pH range.

US-A-4,012,444 and US-A-4,066,755 disclose labetalol hydrochloride, its preparation and uses in the treatment of hypertension.

The present invention is a sustained released preparation for once daily oral administration of labetalol hydrochloride. This oral sustained release dosage form is a tablet containing sufficient labetalol hydrochloride to provide sustained release over a prolonged period contained in a matrix carrier material. The matrix comprises a polymeric binder of hydroxypropylmethylcellulose in combination with polyvinylpyrrolidone, a pharmaceutically acceptable water-soluble organic acid and optionally conventional pharmaceutically acceptable excipients.

The present invention provides a new sustained release tablet of labetalol hydrochloride for once daily administration. In addition to the active ingredient, the tablet comprises a polymeric matrix of hydroxypropylmethylcellulose and polyvinylpyrrolidone and a pharmaceutically acceptable water soluble organic acid. The specific combination of the polymeric matrix and organic acid provides an oral dosage form suitable for once a day dosing.

In accordance with the present invention, the amount of labetalol hydrochloride that is incorporated in a tablet may range between about 200 and about 600 mg. The therapeutic range of about 200-1200 mg per day is indicated for the treatment of hypertension. The tablet of the present invention provides a release period suitable for once daily dosing, i.e., once within a 24 hour period.

The polymeric binder according to the present invention comprises a combination of hydroxypropylmethylcellulose and polyvinylpyrrolidone. The total polymer contents represents 5-20% by weight of the dosage form. A preferred range for the total amount of polymer present in the dosage form is 8-15% by weight. The weight ratio of hydroxypropylmethylcellulose to polyvinylpyrrolidinone in the polymeric binder is 4-2 to 1, preferably 2 to 1.

The hydroxypropylmethylcelluloses/(HPMC) utilized in the present invention are water soluble cellulose ethers, and include, but not limited to, USP 2208, USP 2906 and USP 2910. Examples of such materials are commercially available from Dow Chemical Co. in various grades under several tradenames, including METHOCEL E, (USP 2910), METHOCEL F, (USP 2906) and METHOCEL K, (USP 2208). The various grades differ in methoxy and hydroxypropoxyl content as well as molecular weight and viscosity. Preferred hydroxypropylmethylcellulose polymers useful in carrying out the invention include METHOCEL E4M, characterized by having a 28-30 weight percent methoxyl content, a 7-12 weight percent hydroxypropoxyl content, and a number average molecular weight of 93,000 and a viscosity in a 2% aqueous solution of 3500-5600, cps and METHOCEL K4M having a 19-24 weight percent methoxyl content, a 7-12 weight percent hydroxypropoxyl content, and a number average molecular weight of 89,000, and a viscosity in a 2% aqueous solution of 3500-5600 cps and METHOCEL F4M having a 26-30 weight percent methoxy content, a 4-6 weight percent hydroxypropoxyl content, and a number average molecular weightof 86,000, and a viscosity in a 2% aqueous solution of 3500-5600 cps.

The polyvinylpyrrolidone utilized in the present invention is a synthetic polymer consisting of linear 1-vinyl-2-pyrrolidinone groups in which the degree of polymerization results in polymers of various molecular weights. It is characterized by its viscosity in aqueous solution; relative to that of water, expressed as a K-value, ranging from 10 to 95. An example of a polyvinylpyrrolidinone useful in the formulation of this invention is Povidone USP K29/32 having an average molecular weight of about 40,000 supplied by GAF Corp. Wayne, N.J.

Other polymers which are useful in forming the polymeric binder of the invention include: methyl cellulose, hydroxymethylcellulose, hydroxyethylcellulose, hydroxypropylcellulose, which can be used alone

2

or in combination; carboxymethyl cellulose and the sodium salt thereof, carboxyethyl cellulose and the sodium salt thereof, which can be used alone or in combination; and other hydrocolloids, such as acacia, guar gum. One skilled in the art will known how to select and formulate the above noted materials to be used in the claimed invention.

The organic acids which are effective in the present invention are relatively strong water-soluble pharmaceutically acceptable organic acids. The acids are generally water-soluble aliphatic mono- or polyhydroxy, polycarboxylic acids with a $pK_1$ of 2-4, and preferrably 2-3.5. Representative organic acids include tartaric acid, citric acid, fumaric acid, maleic acid and the like. Preferred organic acids are tartaric acid and citric acid. The amount of organic acid present in the sustained release tablet of this invention represents 20 to 35% by weight of the dosage form. The preferred amount of organic acid is 25-30% by weight of the dosage form.

The tablet also contains one or more lubricating agents, e.g., magnesium stearate, calcium stearate, stearic acid and the like, or mixtures thereof. The lubricating agent is present in an amount of 0.5-3% of the total dosage form weight. A preferred embodiment comprises 1-2% magnesium stearate.

Optionally, the tablet may include minor amounts of other pharmaceutically acceptable excipients such as diluents, binders, coloring agents and flavoring agents.

In preparing the tablets of the invention, conventional tableting techniques are employed, for example dry granulation or wet granulation, and direct compression. One method for manufacturing the tablets involves blending the labetalol hydrochloride, the organic acid and hydroxypropylmethylcellulose in a suitable mixer. The polyvinylpyrrolidone is dissolved in water and the resulting aqueous solution is granulated with the blended material. The granules are dried and milled if necessary. Add any other needed ingredients, for example, lubricant (magnesium stearate), silica gel and the like, and mix. The complete mixture is then subjected to tableting in conventional tableting machines, such as a rotary tablet press, to the desired size and shape. The tablets may be used as is, but are preferably coated by techniques well known in the art.

During accelerated stability testing of the novel tablet of this invention, i.e., 3 months at 45°C, it was observed that the in vitro dissolution stability could be improved by adding the organic acid component in the dry phase of the granulation. The tablet is prepared by mixing the labetalol hydrochloride with the hydroxypropylmethylcellulose and granulate with the aqueous polyvinylpyrrolidone solution. The granules are dried, milled and the organic acid and excipients added and mixed. The mixture is then formed into tablets using conventional techniques.

The following examples describe typical tablet formulations of the sustained release dosage forms of the present invention. The registered trade marks as used in this patent specification are acknowledged as such.

EXAMPLE 1

A sustained release tablet was prepared from the following ingredients:

| Ingredients | mg/tablet |
|---|---|
| Labetalol HCl | 400 |
| Tartaric acid | 210 |
| Hydroxypropylmethylcellulose "E4M" | 40 |
| Polyvinylpyrrolidone | 20 |
| Magnesium stearate | 8 |
| Silica gel | 2 |
| Color solids | 20 |

Manufacture of Tablets

Blend the labetalol hydrochloride, tartaric acid and hydroxypropylmethylcellulose in a suitable mixer. Dissolve the polyvinylpyrrolidone in the water to produce a 75% w/w solution and granulate with the blended material. Dry the granules and mill if necessary. Add the lubricant, magnesium stearate, and silica gel and mix. Compress the mixture on a rotary tablet press to the desired size and shape. The tablets may be coated with standard coating agents using conventional aqueous film-coating procedures if desired.

| Tablet Coating | |
|---|---|
| Ingredients | Approximate mg/tablet |
| Hydroxypropylmethylcellulose (Pharmacoat 606) | 10.64 |
| Polyethylene glycol 3350 NF | 2.64 |
| Methylparaben NF | 0.01 |
| Propylparaben NF | 0.05 |
| Distilled Water | q.s. |
| Coloring agent | 6.64 |
| Carnauba wax | 0.0002 |

Method of Coating

Prepare the polymer solution using standard methods. Combine the polymer solution with the color dispersion and sufficient water. Coat tablets with colored polymer solution and polish the coated tablets using standard procedures.

According to USP XXI dissolution test methods; i.e. one hour in simulated gastric fluid followed by simulated intestinal fluid, the following data was collected:

| In-Vitro Dissolution Time (hours) | Percent Dissolved |
|---|---|
| 1 | 14 |
| 2 | 35 |
| 3 | 51 |
| 4 | 63 |
| 5 | 73 |
| 6 | 81 |
| 7 | 87 |
| 8 | 91 |

EXAMPLE 2

A sustained release tablet was prepared from the following ingredients:

| Ingredients | mg/tablet |
|---|---|
| Labetalol HCl | 400 |
| Tartaric acid | 210 |
| Hydroxypropylmethylcellulose "K4M" | 30 |
| Polyvinylpyrrolidone | 15 |
| Magnesium stearate | 8 |
| Silica gel | 2 |
| Color solids | 20 |

The following results was obtained employing the dissolution test noted in Example 1:

4

| In-Vitro Dissolution Time (hours) | Percent Dissolved |
|---|---|
| 1 | 19 |
| 2 | 46 |
| 3 | 63 |
| 4 | 76 |
| 5 | 86 |
| 6 | 92 |
| 7 | 95 |
| 8 | 96 |

EXAMPLE 3

A sustained release tablet was prepared from the following ingredients:

| Ingredients | mg/tablet |
|---|---|
| Labetalol HCl | 400 |
| Tartaric acid | 210 |
| Hydroxypropylmethylcellulose "E4M" | 30 |
| Polyvinylpyrrolidone | 15 |
| Magnesium stearate | 8 |
| Silica gel | 2 |
| Color solids | 20 |

The following results were obtained employing the dissolution test noted in Example 1:

| In-Vitro Dissolution Time (hours) | Percent Dissolved |
|---|---|
| 1 | 16 |
| 2 | 38 |
| 3 | 59 |
| 4 | 70 |
| 5 | 80 |
| 6 | 87 |
| 7 | 92 |
| 9 | 95 |

EXAMPLE 4

A sustained release tablet was prepared from the following ingredients:

| Ingredients | mg/tablet |
|---|---|
| Labetalol HCl | 400 |
| Citric acid | 200 |
| Hydroxypropylmethylcellulose "K4M" | 30 |
| Polyvinylpyrrolidone | 15 |
| Magnesium stearate | 5 |

The following results were obtained employing the dissolution test noted in Example 1:

| In-Vitro Dissolution Time (hours) | Percent Dissolved |
|---|---|
| 1 | 21 |
| 2 | 47 |
| 4 | 78 |
| 6 | 91 |
| 8 | 94 |

EXAMPLE 5

A sustained release tablet was prepared from the following ingredients:

| Ingredients | mg/tablet |
|---|---|
| Labetalol HCl | 300 |
| Tartaric acid | 156 |
| Hydroxypropylmethylcellulose "E4M" | 40 |
| Polyvinylpyrrolidone | 20 |
| Magnesium stearate | 7 |
| Silica gel | 2 |
| Color solids | 15 |

The following results were obtained employing the dissolution test noted in Example 1:

| In-Vitro Dissolution Time (hours) | Percent Dissolved |
|---|---|
| 1 | 12 |
| 2 | 31 |
| 3 | 45 |
| 4 | 56 |
| 5 | 66 |
| 6 | 74 |
| 7 | 81 |
| 8 | 86 |

EXAMPLE 6

A sustained release tablet was prepared from the following ingredients:

| Ingredients | mg/tablet |
|---|---|
| Labetalol HCl | 200 |
| Tartaric acid | 105 |
| Hydroxypropylmethylcellulose "E4M" | 34 |
| Polyvinylpyrrolidone | 17 |
| Magnesium stearate | 4 |
| Silica gel | 1 |
| Color solids | 10 |

The following results were obtained employing the dissolution test noted in Example 1:

| In-Vitro Dissolution Time (hours) | Percent Dissolved |
|:---:|:---:|
| 1 | 13 |
| 2 | 33 |
| 3 | 47 |
| 4 | 58 |
| 5 | 68 |
| 6 | 75 |
| 7 | 82 |
| 8 | 87 |

EXAMPLE 7

The effect of Tartaric acid on dissolution of Labetalol HCl tablets was studied by preparing tablets containing the following ingredients:

| Ingredients | A mg/tablet | B mb/tablet |
|:---|:---:|:---:|
| Labetalol HCl | 400 | 400 |
| Tartaric acid | 210 | --- |
| Hydroxypropylmethylcellulose "K4M" | 20 | 20 |
| Polyvinylpyrrolidone | 10 | 10 |
| Magnesium stearate | 8 | 8 |
| Silica gel | 2 | 2 |

The following results were obtained employing the dissolution test noted in Example 1:

| In-Vitro Dissolution Time (hours) | Percent Dissolved | |
|:---:|:---:|:---:|
| | A | B |
| 1 | 23 | 16 |
| 2 | 54 | 27 |
| 4 | 87 | 36 |
| 6 | 98 | 39 |
| 8 | 99 | 41 |

EXAMPLE 8

The effect of citric acid on dissolution of Labetalol HCl tablets was studied by preparing tablets containing the following ingredients:

| Ingredients | A mg/tablet | B mb/tablet |
|:---|:---:|:---:|
| Labetalol HCl | 400 | 400 |
| Citric acid | 100 | --- |
| Hydroxypropylmethylcellulose "K4M" | 20 | 20 |
| Polyvinylpyrrolidone | 10 | 10 |
| Magnesium stearate | 3 | 3 |

EP 0 266 707 B1

| In-Vitro Dissolution Time (hours) | Percent Dissolved | |
|---|---|---|
| | A | B |
| 1 | 19 | 18 |
| 2 | 38 | 29 |
| 4 | 61 | 36 |
| 6 | 75 | 37 |
| 8 | 83 | 38 |
| 10 | 88 | 38 |
| 12 | 91 | 38 |

EXAMPLE 9

The effect of addition of tartaric acid - wet phase vs. dry phase - on in vitro accelerated stability of Labelatol HCl tablets was studied by preparing tablets in the following manner:

Formulation A (Wet Phase) - 400 mg. labelatol HCl sustained release tablet was prepared according to the procedure of Example 1 except no coating was applied to the tablet core.

Formulation B (Dry Phase) - 400 mg labelatol HCl sustained release tablet was prepared by mixing and granulating the labelatol HCl and hydroxypropylmethyl cellulose with the aqueous polyvinylpyrrolidone solution. The granules are dried, milled and the excipients (magnesium stearate and silica gel) and the tartaric acid added, mixed and compressed into tablets.

Tablets from Formulations A and B were subjected to accelerated stability testing (3 months at 45°C) and the following results were obtained employing the dissolution test noted in Example 1.

| In-Vitro Dissolution Time (hours) | Percent Dissolved | | | |
|---|---|---|---|---|
| | Formulation A | | Formulation B | |
| | Initial | 3 mo/45°C | Initial | 3 mo/45°C |
| 1 | 15 | 21 | 14 | 18 |
| 2 | 38 | 47 | 33 | 38 |
| 3 | 52 | 64 | 47 | 52 |
| 4 | 64 | 78 | 59 | 64 |
| 5 | 75 | 88 | 69 | 74 |
| 6 | 83 | 93 | 78 | 82 |
| 7 | 89 | 96 | 84 | 88 |
| 8 | 93 | -- | 89 | 92 |

Adding the organic acid, for example tartaric acid, in the dry phase of the granulation procedure results in improved stability for the tablet.

**Claims**
**Claims for the following Contracting States : BE, CH, DE, ES, FR, GB, GR, IT, LI, LU, NL, SE**

1. An oral sustained release dosage unit form tablet comprising a therapeutically effective amount of labetalol hydrochloride; 5-20% by weight of a polymeric binder comprising a combination of hydroxypropylmethylcellulose and polyvinylpyrrolidone in a weight ratio of 4-2 to 1; 20-35% by weight of pharmaceutically acceptable organic acid; and optionally pharmaceutically acceptable excipients.

2. An oral sustaned release dosage unit form tablet comprising a therapeutically effective amount of labetalol hydrochloride; 8-15% by weight of a polymeric binder comprising a combination of hydroxypropylmethylcellulose and polyvinylpyrrolidone; 25-30% by weight of pharmaceutically acceptable organic acid; and 0.5-3% by weight of pharmaceutically acceptable excipients.

8

3. The tablet of claim 1 wherein the polymeric binder comprises a weight ratio of 4 to 1 of hydroxypropylmethylcellulose to polyvinylpyrrolidone.

4. The tablet of claim 1 wherein the polymeric binder comprises a weight ratio of 2 to 1 of hydroxypropylmethylcellulose to polyvinylpyrrolidone.

5. The tablet of claim 1 wherein the pharmaceutically acceptable organic acid is selected from the group consisting of tartaric acid, citric acid, fumaric acid and maleic acid.

6. The oral sustained release tablet of claim 5 wherein the acid is tartaric acid.

7. The oral sustained release tablet of claim 5 wherein the acid is citric acid.

8. The tablet of claim 1 comprising 400 mg of labetalol hydrochloride, 210 mg of tartaric acid, 40 mg of hydroxypropylmethylcellulose, 20 mg of polyvinylpyrrolidone, 8 mg of magnesium stearate and 2 mg silica gel.

9. The tablet of claim 1 comprising 400 mg of labetalol hydrochloride, 210 mg of tartaric acid, 30 mg of hydroxypropylmethylcellulose, 15 mg of polyvinylpyrrolidone, 8 mg of magnesium stearate and 2 mg silica gel.

10. The tablet of claim 1 comprising 300 mg of labetalol hydrochloride, 156 mg of tartaric acid, 40 mg of hydroxypropylmethylcellulose, 20 mg of polyvinylpyrrolidone, 7 mg of magnesium stearate and 2 mg. silica gel.

11. The tablet of claim 1 comprising 200 mg of labetalol hydrochloride, 105 mg of tartaric acid, 34 mg of hydroxypropylmethylcellulose, 17 mg of polyvinylpyrrolidone, 4 mg of magnesium stearate and 1 mg silica gel.

12. A process for preparing a stabilized oral sustained release dosage unit form tablet according to claim 1 which comprises:

   (a) blending labetalol hydrochloride with hydroxypropylmethylcellulose;
   (b) granulating the blended material from (a) with an aqueous solution of polyvinylpyrrolidone;
   (c) adding an organic acid and excipients to the granulate of (b) and mixing the materials
   (d) compressing the mixture of step (c) into tablets.

**Claims for the following Contracting State : AT**

1. A process for preparing a stabilized oral sustained release dosage unit form tablet comprising a therapeutically effective amount of labetalol hydrochloride; 5-20 % by weight of a polymeric binder comprising a combination of hydroxypropylmethylcellulose and polyvinylpyrrolidone in a weight ratio of 4-2 to 1; 20-35 % by weight of pharmaceutically acceptable organic acid; and optionally pharmaceutically acceptable excipients, which comprises:

   (a) blending labetalol hydrochloride with hYdroxypropylmethylcellulose;
   (b) granulating the blended material from (a) with an aqueous solution of polyvinylpyrrolidone;
   (c) adding an organic acid and excipients to the granulate of (b) and mixing the materials
   (d) compressing the mixture of step (c) into tablets.

2. Process according to claim 1 wherein the oral sustained release dosage unit form tablet comprises a therapeutically effective amount of labetalol hydrochloride; 8-15 % by weight of a polymeric binder comprising a combination of hydroxypropylmethylcellulose and polyvinylpyrrolidone; 25-30 % by weight of pharmaceutically acceptable organic acid; and 0.5-3 % by weight of pharmaceutically acceptable excipients.

3. Process according to claim 1 wherein the polymeric binder comprises a weight ratio of 4 to 1 of hydroxypropylmethylcellulose to polyvinylpyrrolidone.

**4.** Process according to claim 1 wherein the polymeric binder comprises a weight ratio of 2 to 1 of hydroxypropylmethylcellulose to polyvinylpyrrolidone.

**5.** Process according to claim 1 wherein the pharmaceutically acceptable organic acid is selected from the group consisting of tartaric acid, citric acid, fumaric acid and maleic acid.

**6.** Process according to claim 5 wherein the acid is tartaric acid.

**7.** Process according to claim 5 wherein the acid is citric acid.

**8.** Process according to claim 1 wherein the tablet comprises 400 mg of labetalol hydrochloride, 210 mg of tartaric acid, 40 mg of hydroxypropylmethylcellulose, 20 mg of polyvinylpyrrolidone, 8 mg of magnesium stearate and 2 mg silica gel.

**9.** Process according to claim 1 wherein the tablet comprises 400 mg of labetalol hydrochloride, 210 mg of tartaric acid, 30 mg of hydroxypropylmethylcellulose, 15 mg of polyvinylpyrrolidone, 8 mg of magnesium stearate and 2 mg silica gel.

**10.** Process according to claim 1 wherein the tablet comprises 300 mg of labetalol hydrochloride, 156 mg of tartaric acid, 40 mg of hydroxypropylmethylcellulose, 20 mg of polyvinylpyrrolidone, 7 mg of magnesium stearate and 2 mg silica gel.

**11.** Process according to claim 1 wherein the tablet comprises 200 mg of labetalol hydrochloride, 105 mg of tartaric acid, 34 mg of hydroxypropylmethylcellulose, 17 mg of polyvinylpyrrolidone, 4 mg of magnesium stearate and 1 mg silica gel.

**Revendications**
**Revendications pour les Etats contractants suivants : BE, CH, DE, ES, FR, GB, GR, IT, LI, LU, NL, SE**

**1.** Comprimé de forme posologigue unitaire à libération prolongée pour administration par voie orale stabilisé comprenant une quantité thérapeutiquement efficace de chlorhydrate de labétalol ; 5 à 20 % en poids d'un liant polymérique comprenant une combinaison d'hydroxypropylméthylcellulose et de polyvinylpyrrolidone dans un rapport pondéral de 4-2 à 1 ; 20 à 35 % en poids d'un acide organique pharmaceutiquement acceptable ; et le cas échéant des excipients pharmaceutiquement acceptables.

**2.** Comprimé à forme posologique unitaire à libération prolongée pour administration par voie orale comprenant une quantité thérapeutiquement efficace de chlorhydrate de labétalol ; 8 à 15 % en poids d'un liant polymérique comprenant une combinaison d'hydroxypropylméthylcellulose et de polyvinylpyrrolidone ; 25 à 30 % en poids d'un acide organique pharmaceutiquement acceptable ; et 0,5 à 3 % en poids d'excipients pharmaceutiquement acceptables.

**3.** Comprimé selon la revendication 1, dans lequel le liant polymérique comprend un rapport pondéral de 4 à 1 de l'hydroxypropylméthylcellulose à la polyvinylpyrrolidone.

**4.** Comprimé selon la revendication 1, dans lequel le liant polymérique comprend un rapport pondéral de 2 à 1 de l'hydroxypropylméthylcellulose à la polyvinylpyrrolidone.

**5.** Comprimé selon la revendication 1, dans lequel l'acide organique pharmaceutiquement acceptable est choisi dans le groupe constitué par l'acide tartrique, l'acide citrique, l'acide fumarique et l'acide maléïque.

**6.** Comprimé à libération prolongée pour voie orale selon la revendication 5, dans lequel l'acide est l'acide tartrique.

**7.** Comprimé à libération prolongée pour administration par voie orale selon la revendication 5, dans lequel l'acide est l'acide citrique.

**8.** Comprimé selon la revendication 1, comprenant 400 mg de chlorhydrate de labétalol, 210 mg d'acide tartrique, 40 mg d'hydroxypropylméthylcellulose, 20 mg de polyvinylpyrrolidone, 8 mg de stéarate de magnésium et 2 mg de gel de silice.

9. Comprimé selon la revendication 1, comprenant 400 mg de chlorhydrate de labétalol, 210 mg d'acide tartrique, 30 mg d'hydroxypropylméthylcellulose, 15 mg de polyvinylpyrrolidone, 8 mg de stéarate de magnésium et 2 mg de gel de silice.

10. Comprimé selon la revendication 1, comprenant 300 mg de chlorhydrate de labétalol, 156 mg d'acide tartrique, 40 mg d'hydroxypropylméthylcellulose, 20 mg de polyvinylpyrrolidone, 7 mg de stéarate de magnésium et 2 mg de gel de silice.

11. Comprimé selon la revendication 1, comprenant 200 mg de chlorhydrate de labétalol, 105 mg d'acide tartrique, 34 mg d'hydroxypropylméthylcellulose, 17 mg de polyvinylpyrrolidone, 4 mg de stéarate de magnésium et 1 mg de gel de silice.

12. Procédé de préparation d'un comprimé à forme posologique unitadre à libération prolongée pour administration orale stabilisé selon la revendication 1, dans lequel :
   (a) on mélange le chlorhydrate de labétalol avec l'hydroxypropylméthylcellulose ;
   (b) on granule la matière mélangée de (a) avec une solution aqueuse de polyvinylpyrrolidone ;
   (c) on ajoute un acide organique et des excipients au granulé de (b) et on mélange les matières ;
   (d) on comprime le mélange de l'étape (c) en comprimés.

**Revendications pour l'Etat contractant suivant : AT**

1. Procédé de préparation d'un comprimé de forme posologique unitaire à libération prolongée pour administration par voie orale stabilisé comprenant une quantité thérapeutiquement efficace de chlorhydrate de labétalol ; 5 à 20 % en poids d'un liant polymérique comprenant une combinaison d'hydroxypropylméthylcellulose et de polyvinylpyrrolidone dans un rapport pondéral de 4-2 à 1 ; 20 à 35 % en poids d'un acide organique pharmaceutiquement acceptable ; et le cas échéant des excipients pharmaceutiquement acceptables, qui consiste :
   (a) à mélanger le chlorhydrate de labétalol avec l'hydroxypropylméthylcellulose ;
   (b) à granuler la matière mélangée de (a) avec une solution aqueuse de polyvinylpyrrolidone ;
   (c) à ajouter un acide organique et des excipients au granulé de (b) et on mélange les matières ;
   (d) à comprimer le mélange de l'étape (c) en comprimés.

2. Procédé selon la revendication 1 dans lequel le comprimé à forme posologique unitaire à libération prolongée pour administration par voie orale comprend une quantité thérapeutiquement efficace de chlorhydrate de labétalol ; 8 à 15 % en poids d'un liant polymérique comprenant une combinaison d'hydrorypropylméthylcellulose et de polyvinylpyrrolidone ; 25 à 30 % en poids d'un acide organique pharmaceutiquement acceptable ; et 0,5 à 3 % en poids d'excipients pharmaceutiquement acceptables.

3. Procédé selon la revendication 1, dans lequel le liant polymérique comprend un rapport pondéral de 4 à 1 de l'hydroxypropylméthylcellulose à la polyvinylpyrrolidone.

4. Procédé selon la revendication 1, dans lequel le liant polymérique comprend un rapport pondéral de 2 à 1 de l'hydroxypropylméthylcellulose à la polyvinylpyrrolidone.

5. Procédé selon la revendication 1, dans lequel l'acide organique pharmaceutiquement acceptable est choisi dans le groupe constitué par l'acide tartrique, l'acide citrique, l'acide fumarique et l'acide maléïque.

6. Procédé selon la revendication 5, dans lequel l'acide est l'acide tartrique.

7. Procédé selon la revendication 5, dans lequel l'acide est l'acide citrique.

8. Procédé selon la revendication 1, dans lequel le comprimé comprend 400 mg de chlorhydrate de labétalol, 210 mg d'acide tartrique, 40 mg d'hydroxypropylméthylcellulose, 20 mg de polyvinylpyrrolidone, 8 mg de stéarate de magnésium et 2 mg de gel de silice.

9. Procédé selon la revendication 1, dans lequel le comprimé comprend 400 mg de chlorhydrate de labétalol, 210 mg d'acide tartrique, 30 mg d'hydroxypropylméthylcellulose, 15 mg de polyvinylpyrrolidone, 8 mg de stéarate de magnésium et 2 mg de gel de silice.

10. Procédé selon la revendication 1, dans lequel le comprimé comprend 300 mg de chlorhydrate de labétalol, 156 mg d'acide tartrique, 40 mg d'hydroxypropylméthylcellulose, 20 mg de polyvinylpyrrolidone, 7 mg de stéarate de magnésium et 2 mg de gel de silice.

11. Procédé selon la revendication 1, dans lequel le comprimé comprend 200 mg de chlorhydrate de labétalol, 105 mg d'acide tartrique, 34 mg d'hydroxypropylméthylcellulose, 17 mg de polyvinylpyrrolidone, 4 mg de stéarate de magnésium et 1 mg de gel de silice.

**Patentansprüche**

**Patentansprüche für folgende Vertragsstaaten : BE, CH, DE, ES, FR, GB, GR, IT, LI, LU, NL, SE**

1. Orale Tabletten in Form von Dosiereinheiten zur Langzeitfreisetzung, umfassend eine therapeutisch wirksame Menge an Labetalol-hydrochlorid; 5-20 Gew.-% eines polymeren Bindemittels, umfassend eine Kombination von Hydroxypropylmethylcellulose und Polyvinylpyrrolidon in einem Gewichtsverhältnis von 4-2 zu 1; 20-35 Gew.-% einer pharmazeutisch annehmbaren organischen Säure; und gegebenenfalls pharmazeutisch annehmbare Trägerstoffe.

2. Orale Tabletten in Form von Dosiereinheiten zur Langzeitfreisetzung, umfassend eine therapeutisch wirksame Menge an Labetalol-hydrochlorid; 8-15 Gew.-% eines polymeren Bindemittels, umfassend eine Kombination von Hydroxypropylmethylcellulose und Polyvinylpyrrolidon; 25-30 Gew.-% einer pharmazeutisch annehmbaren organischen Säure; und 0,5-3 Gew.-% pharmazeutisch annehmbare Trägerstoffe.

3. Tablette nach Anspruch 1, worin das polymere Bindemittel ein Gewichtsverhältnis von 4 zu 1 an Hydroxypropylmethylcellulose zu Polyvinylpyrrolidon umfaßt.

4. Tablette nach Anspruch 1, worin das polymere Bindemittel ein Gewichtsverhältnis von 2 zu 1 an Hydroxypropylmethylcellulose zu Polyvinylpyrrolidon umfaßt.

5. Tablette nach Anspruch 1, wobei die pharmazeutisch annehmbare organische Säure ausgewählt ist aus der Gruppe bestehend aus Weinsäure, Citronensäure, Fumarsäure und Maleinsäure.

6. Oraltablette mit Langzeitfreisetzung nach Anspruch 5, worin die Säure Weinsäure ist.

7. Oraltablette mit Langzeitfreisetzung nach Anspruch 5, worin die Säure Citronensäure ist.

8. Tablette nach Anspruch 1, umfassend 400 mg Labetalolhydrochlorid, 210 mg Weinsäure, 40 mg Hydroxypropylmethylcellulose, 20 mg Polyvinylpyrrolidon, 8 mg Magnesiumstearat und 2 mg Kieselgel.

9. Tablette nach Anspruch 1, umfassend 400 mg Labetalolhydrochlorid, 210 mg Weinsäure, 30 mg Hydroxypropylmethylcellulose, 15 mg Polyvinylpyrrolidon, 8 mg Magnesiumstearat und 2 mg Kieselgel.

10. Tablette nach Anspruch 1, umfassend 300 mg Labetalolhydrochlorid, 156 mg Weinsäure, 40 mg Hydroxypropylmethylcellulose, 20 mg Polyvinylpyrrolidon, 7 mg Magnesiumstearat und 2 mg Kieselgel.

11. Tablette nach Anspruch 1, umfassend 200 mg Labetalolhydrochlorid, 105 mg Weinsäure, 34 mg Hydroxypropylmethylcellulose, 17 mg Polyvinylpyrrolidon, 4 mg Magnesiumstearat und 1 mg Kieselgel.

12. Verfahren zur Herstellung einer stabilisierten oralen Formtabletten-Dosiereinheit zur Langzeitfreisetzung nach Anspruch 1, umfassend:

    (a) Vermischen von Labetalol-hydrochlorid mit Hydroxypropylmethylcellulose;
    (b) Granulieren des vermischten Materials aus (a) mit einer wäßrigen Lösung von Polyvinylpyrrolidon;

(c) Zugabe von organischer Säure und Trägerstoffen zum Granulat von (b) und Mischen der Materialien;

(d) Pressen der Mischung von Schritt (c) zu Tabletten.

**Patentansprüche für folgenden Vertragsstaat : AT**

1. Verfahren zur Herstellung stabilisierter oraler Tabletten in Form von Dosiereinheiten zur Langzeitfreisetzung, umfassend eine therapeutisch wirksame Menge an Labetalol-hydrochlorid; 5-20 Gew.-% eines polymeren Bindemittels, umfassend eine Kombination von Hydroxypropylmethylcellulose und Polyvinylpyrrolidon in einem Gewichtsverhältnis von 4-2 zu 1; 20-35 Gew.-% einer pharmazeutisch annehmbaren organischen Säure; und gegebenenfalls pharmazeutisch annehmbare Trägerstoffe, welches umfaßt:

   (a) Vermischen von Labetalol-hydrochlorid mit Hydroxypropylmethylcellulose;
   (b) Granulieren des vermischten Materials aus (a) mit einer wäßrigen Lösung von Polyvinylpyrrolidon;
   (c) Zugabe von organischer Säure und Trägerstoffen zum Granulat von (b) und Mischen der Materialien;
   (d) Pressen der Mischung von Schritt (c) zu Tabletten.

2. Verfahren nach Anspruch 1, wobei die oralen Tabletten in Form von Dosiereinheiten zur Langzeitfreisetzung eine therapeutisch wirksame Menge an Labetalol-hydrochlorid; 8-15 Gew.-% eines polymeren Bindemittels, umfassend eine Kombination von Hydroxypropylmethylcellulose und Polyvinylpyrrolidon; 25-30 Gew.-% einer pharmazeutisch annehmbaren organischen Säure; und 0,5-3 Gew.-% pharmazeutisch annehmbarer Trägerstoffe umfassen.

3. Verfahren nach Anspruch 1, wobei das polymere Bindemittel ein Gewichtsverhältnis von 4 zu 1 an Hydroxypropylmethylcellulose zu Polyvinylpyrrolidon umfaßt.

4. Verfahren nach Anspruch 1, wobei das polymere Bindemittel ein Gewichtsverhältnis von 2 zu 1 an Hydroxypropylmethylcellulose zu Polyvinylpyrrolidon umfaßt.

5. Verfahren nach Anspruch 1, wobei die pharmazeutisch annehmbare organische Säure ausgewählt ist aus der Gruppe bestehend aus Weinsäure, Citronensäure, Fumarsäure und Maleinsäure.

6. Verfahren nach Anspruch 5, wobei die Säure Weinsäure ist.

7. Verfahren nach Anspruch 5, wobei die Säure Citronensäure ist.

8. Verfahren nach Anspruch 1, wobei die Tablette 400 mg Labetalol-hydrochlorid, 210 mg Weinsäure, 40 mg Hydroxypropylmethylcellulose, 20 mg Polyvinylpyrrolidon, 8 mg Magnesiumstearat und 2 mg Kieselgel umfaßt.

9. Verfahren nach Anspruch 1, wobei die Tablette 400 mg Labetalol-hydrochlorid, 210 mg Weinsäure, 30 mg Hydroxypropylmethylcellulose, 15 mg Polyvinylpyrrolidon, 8 mg Magnesiumstearat und 2 mg Kieselgel umfaßt.

10. Verfahren nach Anspruch 1, wobei die Tablette 300 mg Labetalol-hydrochlorid, 156 mg Weinsäure, 40 mg Hydroxypropylmethylcellulose, 20 mg Polyvinylpyrrolidon, 7 mg Magnesiumstearat und 2 mg Kieselgel umfaßt.

11. Verfahren nach Anspruch 1, wobei die Tablette 200 mg Labetalol-hydrochlorid, 105 mg Weinsäure, 34 mg Hydroxypropylmethylcellulose, 17 mg Polyvinylpyrrolidon, 4 mg Magnesiumstearat und 1 mg Kieselgel umfaßt.